# EUROPEAN PATENT SPECIFICATION

(11) **EP 1 956 992 B1**
(45) Date of publication and mention of the grant of the patent: **06.03.2013**
(21) Application number: 06821453.5
(22) Date of filing: 15.11.2006
(51) Int. Cl.: A61B 18/14, A61B 18/12, A61M 25/01

(54) **AUTOMATING THE ABLATION PROCEDURE TO MINIMIZE THE NEED FOR MANUAL INTERVENTION**
AUTOMATISIERUNG EINES ABLATIONSVERFAHRENS ZUR MINIMIERUNG DER NOTWENDIGKEIT MANUELLER EINGRIFFE
AUTOMATISATION DE LA PROCEDURE D'ABLATION POUR MINIMISER LE RECOURS A UNE INTERVENTION MANUELLE

(30) Priority: 02.12.2005 US 741740 P
(43) Date of publication of application: 20.08.2008
(73) Proprietor: Koninklijke Philips Electronics N.V., 5621 BA Eindhoven (NL)
(72) Inventor: GROTH, Alexandra, NL-5621 BA Eindhoven (NL); ECK, Kai, NL-5621 BA Eindhoven (NL)
(74) Representative: Verweij, Petronella Danielle
(86) International application number: PCT/IB2006/054269
(87) International publication number: WO 2007/063443

(56) References cited:
- EP-A- 1 554 986
- WO-A-00/16684
- WO-A-00/69353
- WO-A-01/21253
- WO-A-01/58373
- WO-A-02/32335
- WO-A-02/38064
- US-A- 6 035 226
- US-A- 6 063 022
- US-A1- 2002 019 644
- US-A1- 2002 072 662

## Description

The present invention relates to ablation of body tissue and, more particularly, to automating the various stages of the procedure.

Ablation is a therapeutic procedure iteratively performed, point-by-point, to destroy those points or sites in the selected portion of body tissue, such as in removing a tumor. Ablation can also be applied to prevent an abnormal electrical signal from traversing the heart -- electrical signals normally travel across the heart to maintain the heart beat, but an existing abnormal signal may cause an abnormal heart beat rhythm, i.e., arrhythmia. Radiofrequency (RF) ablation is an ablation technique that enjoys a high success rate with low incidence of complications.

For every cardiac arrhythmia, there is an anatomic region of abnormal pulse generation or propagation. If this region is irreversibly destroyed by a catheter ablation, the arrhythmia disappears by virtue of the electrical conduction becoming blocked. RF cardiac catheter ablation introduces catheters intravenously to contact a selected area inside the heart. Among the catheters are an ablation catheter, and other catheters for taking measurements and delivering pulses. The catheter tip contains one or more electrodes. An alternating current is produced at radio frequency, e.g., from about 300 to 750 kHz. The current is typically delivered for about 10 to 60 seconds at a time. The rapidly changing electric field causes ions at the tip of the electrode to rapidly alternate position. Resulting friction heats the body tissue at the ablation catheter tip. By controlling electrical parameters of the circuit, the heating can be kept at a level, generally about 65° C, which "cooks" or ablates the targeted tissue to cause a lesion at that location. The temperature to which the tissue is heated rapidly decreases with distance from the tip. Thus, when one spot is ablated, the tip is steered and moved to a next adjoining spot if a contiguous region is to be ablated. The process repeats, until the entire region or, as typically the case in treating arrhythmia, the entire line or ring is ablated. Due to the efficiency of RF ablation, in comparison with, for example, direct current (DC) ablation, the procedure is relatively painless, and the patient is conscious through the procedure.

To prevent gaps in the ablation which might allow harmful electrical conduction to penetrate, the specific ablation points in a pre-planned ablation path are, point-wise, densely ablated for a distance along the path sufficient to block the electrical conduction. The path may be planned by conducting an electrophysiological (EP) study. The EP study can involve introducing catheters into the heart intravenously, guiding the catheters to particular locations and taking measurements based on readings or samples from the catheters. Ablation need not occur at the EP stage, but typically would occur later, once the EP map is fully developed. Then, the interventionalist decides by looking at the EP map and at other available information (e.g., CT) where to ablate. However, although the interventionalist mentally combines this information, it is not registered or combined by systematic technical approaches to optimize and predict the outcome. The pre-planned path, however, though perhaps visible on a computer screen, is not recorded for subsequent automatic execution of the ablation.

Although, for a favorable intervention outcome, precise ablation path planning and densely sampled pathways are essential, the electrophysiologist, who is a specially trained cardiologist, has to count mainly on his or her expert knowledge during the ablation procedure. In particular, nearly no quality assuring support is provided in the different stages of ablation. For example, the clinician is not assisted by a rule-based system in planning the ablation path beforehand. The consequences of a planned path are not cross-checked with other information that is typically available, therefore potentially leading to on-the-fly decisions during ablation or suboptimal results. Nor is the planned path recorded.

Also, since not all information is taken into account and not all consequences of the chosen path are taken into account, path planning is imprecise, and the selected path might not be the best solution that is possible. As a result, re-ablation of paths is often required to create an uninterrupted line of ablation that prevents an errant component of the signal propagation through the heart from crossing the ablation line. Likewise, an additional ablation path may be needed to obtain a sufficient result, as a result of signal propagation not having changed to the desired behavior. During ablation, the clinician monitors parameters such as temperature and power, manually controls the duration of ablation at each point, and manually steers the ablation catheter from point to point. Since steering and lesion forming are manual procedures, the catheter ablation technique is laborious and time consuming

In today's approach, the average ablation procedure takes about two hours, and the quality of the outcome depends strongly on the know-how of the electrophysiologist.

Document WO 02138064 discloses an ablation apparatus according to the preamble of claim 1.

There exists a need to reduce the duration of imaging when X-rays are involved and to increase patient throughput. There also exists a need to make ablation more precise, according to the best solution under the given boundary conditions. There likewise exists a need to simplify the ablation procedure, especially for less experienced physicians.

In accordance with the invention, an ablation apparatus is presented, wherein the ablation apparatus comprises:
means for selecting a plurality of points that reside along a predetermined ablation path in tissue of a body, and saving the selected points;
means for determining, automatically and without user intervention, when ablation is completed at a current point of the plural points; and
means for, upon said determining of completion, steering, automatically and without user intervention, from said current point to a next of the plural points;
wherein the ablation apparatus is characterized by further comprising
means for executing, automatically and without user intervention, for the plural points, each of the following steps a) through d):
a) steering an ablation device to approach a current one of the points for commencement of ablation at the current point when said current point is reached;
b) determining whether said approach is unsuccessful, and, if so, storing a location of said current point;
c) if it is determined that said approach is successful, determining whether said ablation at said current point is unsuccessful, and, if so, storing said location; and
d) repeating steps a) through c) for a next point, until a last point is processed.

As set forth hereinbelow, it is proposed to execute all ablation steps in succession with the smallest amount of direct intervention as possible. The novel ablation process preferably includes: planning, to derive a path; automatically steering to ablation points on the derived path to arrive at said points; automatically controlling ablation at the arrived-at points based on parameters that vary during the ablation; automatically determining ones of the arrived-at points at which the ablation has failed; automatically recording locations of the arrived-at points where it is determined that ablation has failed; and performing functional outcome control that automatically approaches, in succession, new ablation points arising from the outcome control.

In another aspect of the present invention, an electrophysiological (EP) map or study is made of heart that is to undergo ablation. Morphology of the patient's heart is analyzed to form a heart model. Specifications are provided of a catheter system to be used in the ablation. An optimal ablation path is then created, automatically and without user intervention, based on the map and subject to the formed model and the provided specifications. The resulting optimal path is recorded, automatically and without user intervention.

In a further aspect, points that reside along a predetermined ablation path in tissue of a body are selected and saved. For a current one of the saved points, a determination is made as to whether ablation at that point is completed. The determination is made automatically and without user intervention. At the time of completion, the ablation device is steered, automatically and without user intervention, to move within the body from the current point to a next point.

In yet another aspect, parameters are monitored during ablation at a point. It is determined, automatically and without user intervention, when ablation at that point is completed. Ablating to completion is performed subject to termination of the ablating, automatically and without user intervention, before completion at that point based on the monitored parameters. For example, checking may be done as to whether a catheter performing the ablation loses physical contact with body tissue undergoing the ablation at a site and as to whether a temperature at the site falls below a predetermined threshold. In either situation, ablation at the point is terminated. Automatic monitoring is also performed to detect any condition that might indicate danger to the patient, and the ablation is halted automatically and immediately upon detecting such a condition.

Details of the invention are set forth below with the aid of the following drawings, wherein:
FIG. 1 is a block diagram of components of an exemplary, integrated electrophysiology (EP) workstation requiring minimal manual intervention, according to the present invention;
FIG. 2 is a flow chart serving as an overview of a cardiac ablation procedure according to the present invention;
FIG. 3 is a flow chart of an example of a cardiac ablation preparation process according to the present invention;
FIG. 4 is a flow chart of one embodiment of a cardiac ablation process, according to the present invention;
FIG. 5 is a flow chart of an exemplary post-ablation outcome control procedure according to the present invention;
FIG. 6 is a flow chart setting forth examples of checks made during ablation, according to the present invention; and
FIG. 7 is a flow chart of processing in a particular embodiment for steering and movement of a catheter in accordance with the present invention.

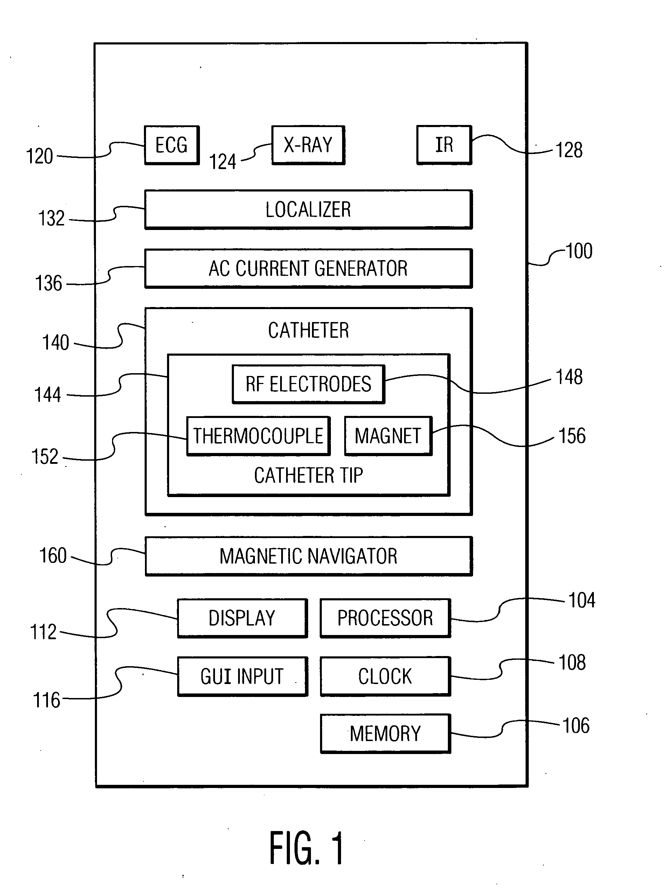

FIG. 1 depicts, by way of illustrative and non-limitative example, an exemplary, integrated electrophysiology (EP) workstation 100 requiring minimal manual intervention, according to the present invention. The workstation 100 has a processor 104 including storage memory 106, a clock 108, a workstation display 112 and a graphical user interface (GUI) input device 116. The workstation 100 further includes an electrocardiogram unit 120, an X-ray or MR or other modality unit 124, an infrared camera 128, a three-dimensional (3D) anatomic catheter localizer 132, and an AC current generator 136. An ablation catheter 140 has a tip 144 that incorporates radio-frequency (RF) AC electrodes 148 for ablating, a thermocouple or thermistor 152 for monitoring temperature at the current ablation point or site, and a magnet 156 by which to magnetically steer the catheter 140. A magnetic navigator 160 steers the tip 144 by means of its magnet 156. Although merely the ablation catheter is shown, it is understood that a number of catheters may be, and typically would be, utilized. A measuring catheter measures electrical activity during the EP study, and may be accompanied by a reference catheter. Pacing catheters are usable to deliver and receive pulses to artificially pace the heart and provide the possibility of testing the integrity of an ablation line, i.e., line of ablation points formed to block errant electrical conduction across the heart.

Although the present invention is described herein in the context of RF ablation, it is within the intended scope of the invention to alternatively employ other ablation technologies, such as direct current, laser, ultrasound, cryothermy, microwave and alcohol.

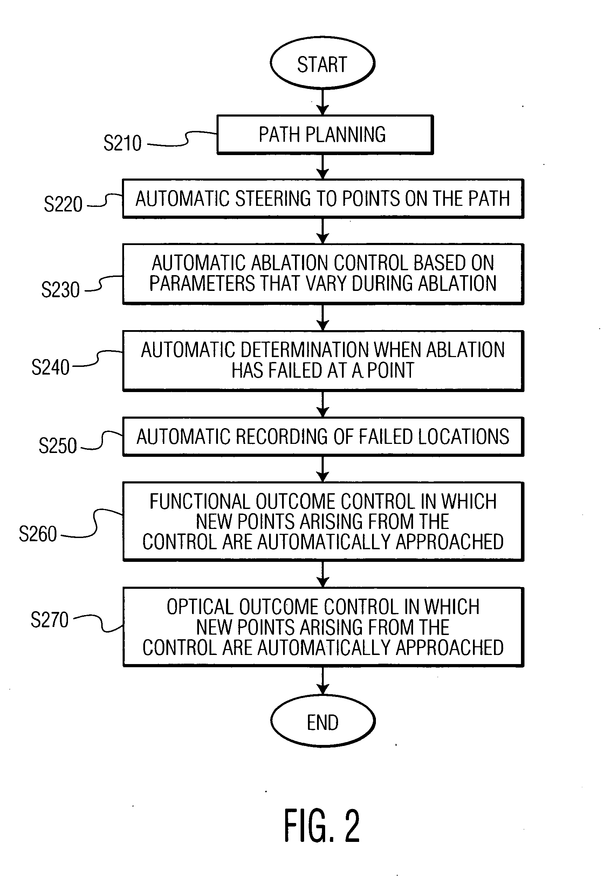

FIG. 2 provides an overview of a novel method for ablation according to the present invention. An ablation path is pre-planned (step S210). During the ablation, steering to points on the path is automatic (step S220). The ablation is automatically controlled based on parameters that vary during the ablation (step S230). Failure of the ablation at a point is automatically determined (step S240). The location of each failed point is automatically recorded (step S250). Functional output control is performed, and new points arising from the control are automatically approached for ablation (step S260). Optical output control may optionally be executed. New points arising from the optical output control are automatically approached for ablation (step S270).

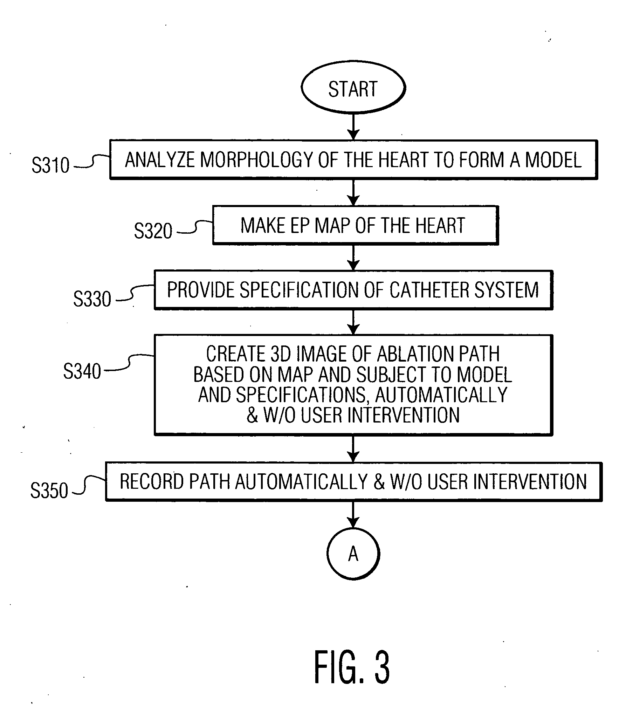

FIG. 3 shows a preferred embodiment of a cardiac ablation preparation process 200 according to the present invention, and corresponds to step S210.

Preliminarily, a non-patient-specific model of the heart may be formed that incorporates a priori knowledge about the physiology of the heart. This preliminary model is then adapted to the patient based on the results of imaging scans. The model offers the advantage of reducing the number of measurements and, more importantly, affording prediction of electrophysiological changes likely to result from the upcoming ablation procedure. Different models, that model the properties of the heart to a different degree, are possible. Some examples are electrophysiological models, electro-anatomical models, and models that comprise the mechanical properties based on a priori knowledge and the current input information available.

A morphological 3D data set is acquired, as by taking a computed tomography (CT) scan of the patient's heart, to form a model of the heart (step S310). To this aim, the heart might be segmented in the 3D data set. The CT scan can be ECG-gated for imaging different contraction statuses of the heart, so that movement of the heart can be made part of the model. Other imaging modalities, such as magnetic resonance (MR) imaging and X-ray volume imaging may be employed instead of the CT scan in acquiring the data set.

Also, an EP study is undertaken (step S320), and involves positioning electrodes in the heart to measure and record electrical activity. The catheters 140 are guided intravenously by the magnetic navigator 160 to selected locations in the heart, and the measuring and reference electrodes take samples of the electrical activity. These samples may be time-coordinated to the phases of the heart beat, to collect information by phase. An EP map is thereby developed. The anatomic catheter localizer 132 detects merely relative position of the catheter tip 144, and is accordingly registered to the 3D data set to then update the model formed in step S310.

Based on the EP map and the heart model, cardiac regions that may be inaccessible or unreachable due to individual patient peculiarities are excluded in the path planning process, in advance of the actual ablating. The EP map might show necrotic tissue which does not conduct signals, and therefore can be excluded from the planned path. In addition to these considerations, catheter system specifications are needed for steering the catheter 140 (step S330). For example, certain regions of the heart may be inaccessible due to the shape of the catheter. The processor 104 creates an optimal ablation path automatically, and without user intervention, based on predictions of the model, which, in turn, are based on the EP map, and subject to heart model and catheter specifications (step S340). The processor 104 can alternatively choose the best ablation paths by testing a set of given ablation paths and deciding in favor of the path with the best predicted outcome. In a preferred embodiment, a small set of predetermined ablation paths, e.g., 30 paths, is subject to a full search for the optimal path given the boundary conditions afforded by the map, model and specifications.

Advantageously, the path is recorded as a list of point coordinates, or, e.g., as a 3D image, into the memory 106, automatically and without user intervention, for subsequent selection of points to be ablated (step S350). Accordingly, the novel path planning procedure is an iterative approach that exploits all available information and crosschecks it to provide the electrophysiologist with the optimal ablation path for each individual patient and catheter system 140.

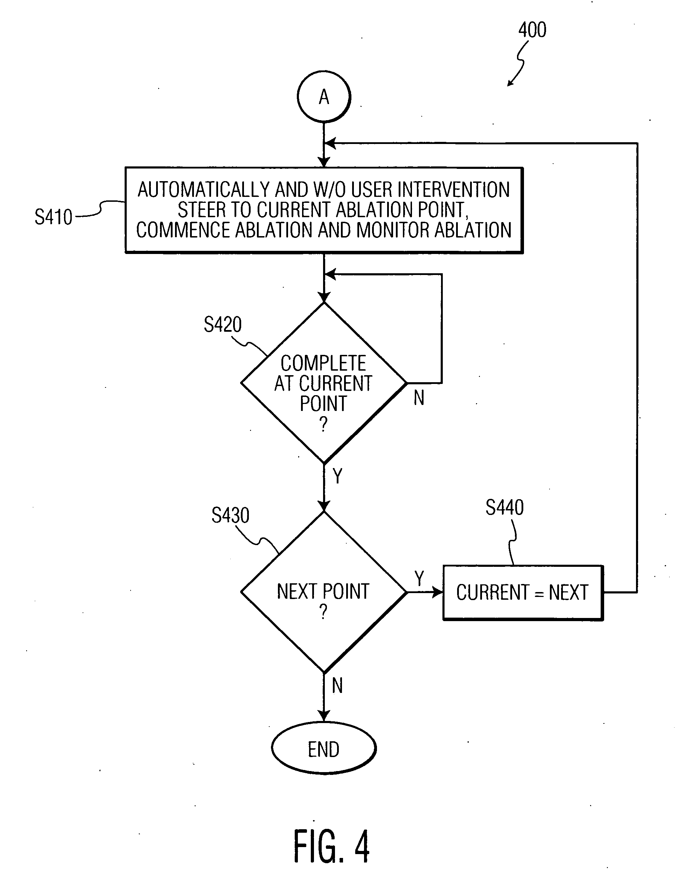

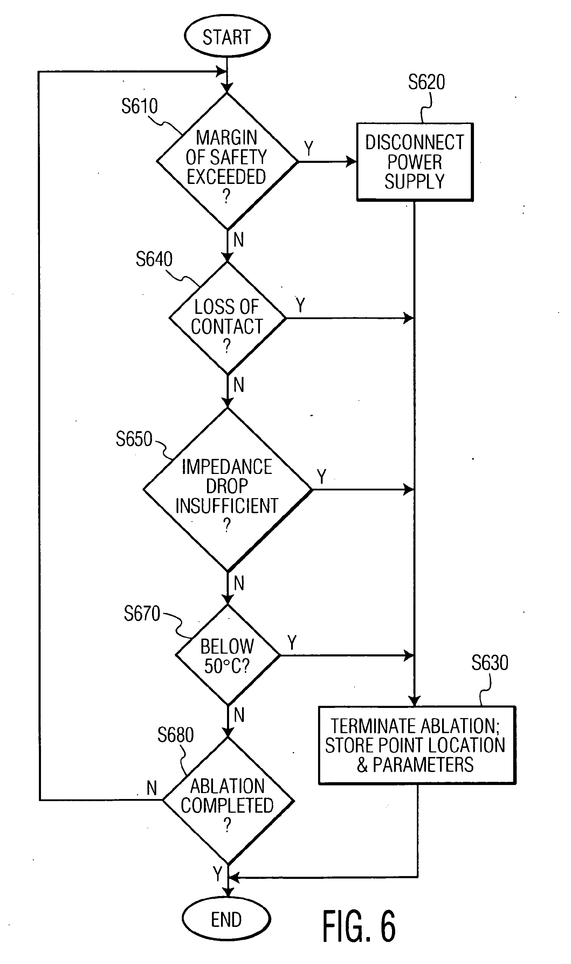

FIG. 4 provides details of an exemplary cardiac ablation process 400, according to the present invention. The previously-calculated optimal ablation path is retrieved from memory 106. The magnetic navigator 160 is operable, in conjunction with the anatomic catheter localizer 132, to, automatically and without user intervention, steer the ablation catheter tip 144 to the current ablation point, so that ablation can commence at that current point while operating parameters of the ablation are automatically monitored (step S410). Electric current from an electrode 148 passes through the patient's body to return to the generator 136 by means of a patch electrode on the patient's chest. The magnetic navigator 160 creates a magnetic field to steer the catheter tip 144, by means of its magnet 156, to the first point. Position measurement to detect arrival at the first point is accomplished by the localizer 132, which may be a system such as LocaLisa^{™} or Real time Position Management^{™}. Alternatively, the catheter position can be extracted from real time image data such as an X-ray or fluoroscopic image. Once arrival is detected, the catheter electrode tip 144 is automatically, and without user intervention, activated to start ablation that ablates body tissue within a small radius. When the ablation is complete because a sufficient lesion has been formed, as determined by the processor 104 based on its reading of system parameters, the RF energy delivery is halted. This determination is made, preferably without the need for user interaction, based upon system parameters that are monitored and a set of rules for determining completion at the current point. Ablation duration at a point is typically from 10 to 60 seconds. One parameter that may be monitored is the power output, typically 20 to 50 watts. The temperature, may be kept constant, e.g., at about 65° C, by feedback from the thermocouple or thermistor 152, or may be allowed to vary. Impedance is another parameter that may be monitored. When ablation is complete at the current point (step S420), or when ablation at the current point fails and is interrupted, as discussed below in conjunction with FIG. 6, a next point along the ablation path is selected if a next point remains (step S430, S440). Otherwise, if no next point along the ablation path remains, any failed and early terminated point is revisited as the next point (step S440). An alternative to the revisit is a semi-manual procedure by the interventionalist to successfully ablate the failed point, or a completely manual procedure. When no point for ablation remains, because each point has been successfully ablated, thereby completing the ablation line, function outcome control begins. As an alternative to the above-mentioned revisiting after a first pass through the ablation line, the failed point may be revisited immediately.

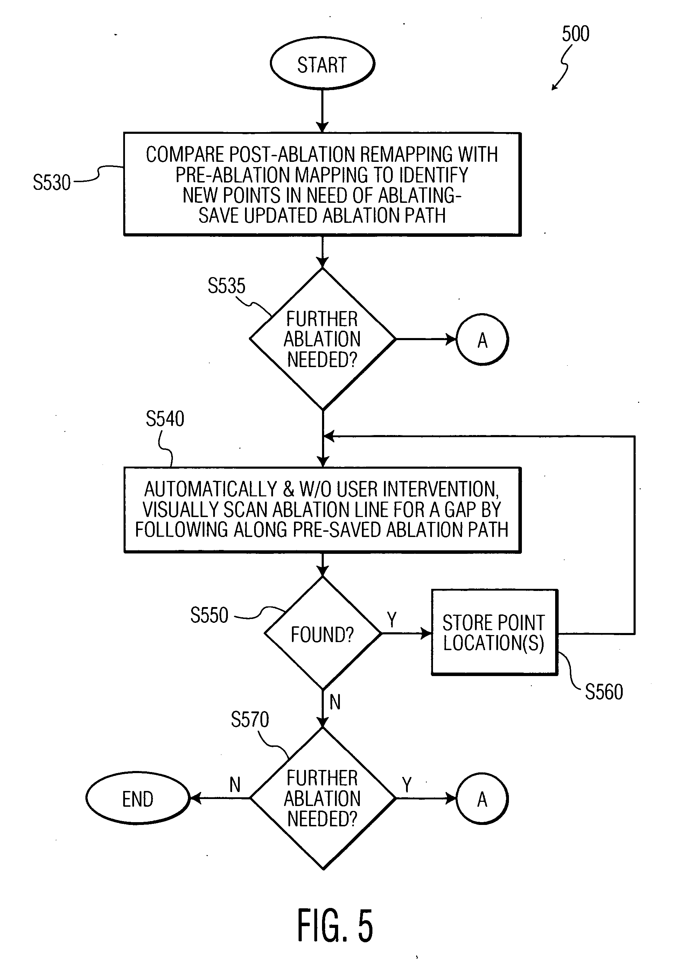

FIG. 5 demonstrates an example of a preferred post-ablation outcome control procedure 500 according to the present invention.

Functional outcome control intends to cover, for example, the possibility that the scar previously formed in the ablation process 400 is insufficiently deep within the heart tissue to totally prevent propagation of an errant signal. In the functional outcome control procedure 500, when the ablation line is completed (step S430, "NO" branch), a post-ablation remapping is performed by guiding a catheter, in the same manner the pre-ablation mapping was performed. A comparison of the two mappings is made to identify new points in need of ablation. For functional outcome control, pacing by means of the pacing electrodes, i.e., sending a signal across the ablation line from one electrode to the other, may also be used to identify new points. The new points are saved electronically to memory 106 as an updated ablation path (step S530). If new points have been identified, so that further ablation is necessary (step S535), the cardiac ablation process 400 is re-executed for these points. Automation of functional outcome control includes automating, according to the cardiac model, the determination of where to place the pacing electrodes.

In the subsequent, optical outcome control procedure, the infrared camera 128 automatically, and without user intervention, completes a visual scan of the ablation line to find gaps (step S540). In performing the scan, the ablation path pre-saved in step S530 is followed. If a gap is found (step S550), the scan is paused, and the point location(s) are stored in memory 106 (step S560). The scan then resumes at step S540. If no gaps are found, or if no further gaps are found (step S550), query is made as to whether further ablation is needed (step S570). If not, the process 500 is complete. Otherwise, processing returns to step S410 on a next ablation pass, this one devoted to filling the gaps.

FIG. 6 illustrates an example of an ablation interruption process 600 according to the present invention. The interruption process 600 operates during step S410 to selectively interrupt ablation upon occurrence of a predetermined condition. If monitored parameters exceed the set margin of safety (step S610), the power supply is automatically disconnected (step S620). Ablation at the current point is terminated, and the point location and current parameters are stored (step S630). As another condition, if loss of contact is detected (step S640), ablation at the current point is terminated and parameters are stored (step S630). Loss of contact can be detected by a motion pattern of the catheter tip using Fourier methods. It can also be detected by a rise in current usage if temperature is being maintained to a pre-set level, e.g., at least 50° C, because the patient's bloodstream is acting as heat sink. If temperature is not being maintained within a set range, loss of contact is immediately noticeable from a fast drop in temperature, due to the heat sink effect. Another condition would be an insufficient drop in capacitive impedance (step S650), indicating that ablation is not successful at the current point. Application of the AC current causes a rise in frequency that would normally lower capacitive impedance. The failure of the impedance to drop, by about 10% within a predefined ablation duration, might be caused by the formation of coagulum on the catheter tip 144. Finally, if the temperature is below 50° C (step S670), loss of contact is presumed due to the above-described "heat sink" effect. If none of the conditions exists, and ablation for the current point is ongoing (step S680), return is made to repeat the checks (step S610).

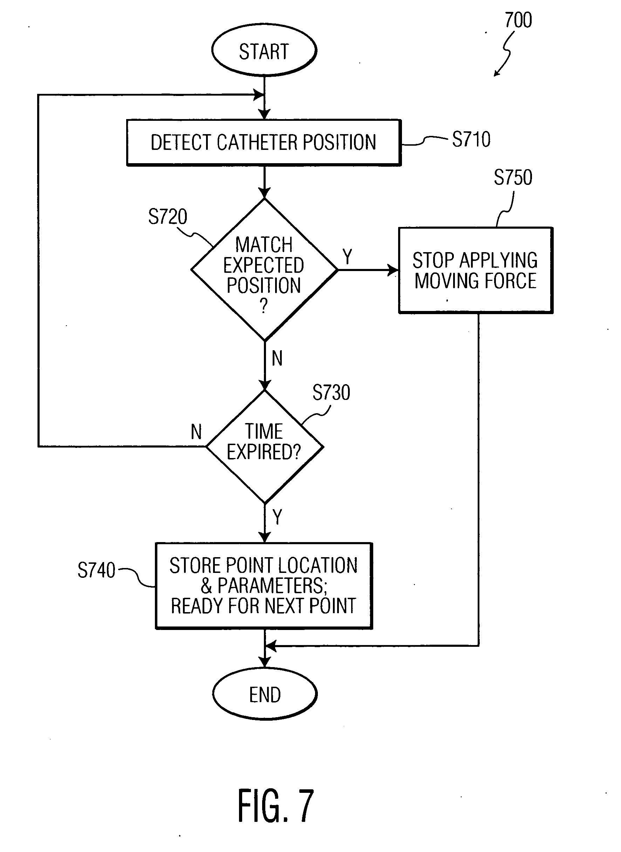

FIG. 7 shows an exemplary process 700 for steering the catheter tip 144 according to the present invention. The process 700 operates concurrently with step S410. The anatomic catheter localizer 132 monitors the current position of the catheter tip 144 by comparing the position, in real-time, to an expected position. The expected position is that of the next, i.e., destination, ablation point, and is accessible from the stored 3D image or list of ablation point coordinates. Accordingly, the localizer 132 detects the location of the catheter tip 144 (step S710), checks as to whether it matches with an expected position (step S720) and, if not, determines whether the time for maneuvering to the next point has expired (step S730). If the time has not expired, the process repeats starting at step S710. Otherwise, if the time has expired (step S730), steering to the next point has timed out. The location of the next point, i.e., the attempted destination, and the current parameters are stored, and the following point becomes the present destination (step S740). If, on the other hand, comparison of the catheter tip's present location and the expected location leads to the conclusion that the expected location has been reached, the magnetic navigator holds the catheter tip position until the ablation is finished (step S750).

While there have been shown and described and pointed out fundamental novel features of the invention as applied to preferred embodiments thereof, it will be understood that various omissions and substitutions and changes in the form and details of the devices illustrated, and in their operation, may be made by those skilled in the art. For example, the optical outcome procedure may be foregone to save time, since functional outcome control may suffice. It should be recognized that structures and/or elements and/or method steps shown and/or described in connection with any disclosed form or embodiment of the invention may be incorporated in any other disclosed or described or suggested form or embodiment as a general matter of design choice.

## Claims

1. An ablation apparatus, comprising:
means (120, 104, 106) for selecting a plurality of points that reside along a predetermined ablation path in tissue of a body, and saving the selected point (S210);
means (152) for determining, automatically and without user intervention, when ablation is completed at a current point of the plural points; and
means (160) for, upon said determining of completion, steering, automatically and without user intervention, from said current point to a next of the plural points (S420);
wherein the ablation apparatus is **characterized by** further comprising
means (104) for executing, automatically and without user intervention, for the plural points, each of the following steps a) through d):
a) steering an ablation device to approach a current one of the points for commencement of ablation at the current point when said current point is reached (S410);
b) determining whether said approach is unsuccessful, and, if so, storing a location of said current point (S740);
c) if it is determined that said approach is successful, determining whether said ablation at said current point is unsuccessful, and, if so, storing said location (S630); and
d) repeating steps a) through c) for a next point, until a last point is processed (S430).

2. The ablation apparatus of claim 1, further comprising means (120, 104) detecting a predetermined condition, automatically and without user intervention, and means for, upon such detecting, a) interrupting said ablation at said current point, b) moving said device to said next of the plural points, and c) performing ablation at said next point.

3. The ablation apparatus of claim 1, further comprising:
means (120) for making an electrophysiological map of a heart that is to undergo ablation;
means (104) for at least one of analyzing morphology of said heart to form a heart model and providing specifications of a catheter system to be used in said ablation, and for creating an ablation path, based on the made map and subject, correspondingly, to at least one of the formed model and the provided specifications; and
means (106) for recording the formed path.

4. The ablation apparatus of claim 1, further comprising:
means (152) for detecting that a catheter (140) performing said ablation has lost physical contact with body tissue or that a temperature at said point has fallen below a predetermined threshold; and
means (104) for terminating ablation if either situation occurs.

5. The ablation apparatus of claim 1, further comprising:
means (144) for checking whether impedance encountered by an electric circuit drops below a predefined threshold within a pre-set ablation duration (S650); and
means (104) for terminating said ablation at said point if impedance is determined to not drop by said predefined threshold within the pre-set duration (S630).

6. The ablation apparatus of claim 1, further comprising means (104) for comparing a post-ablation remapping with a pre-ablation mapping, said arising resulting from said comparing.

7. The ablation apparatus of claim 1, further comprising optical output control (128) for visually scanning along a path that includes said new ablation points.

## Patentansprüche

1. Ablationsgerät, das Folgendes umfasst:
Mittel (120, 104, 106) zum Auswählen einer Vielzahl von Punkten, die entlang eines vorgegebenen Ablationspfads im Gewebe eines Körpers liegen, und zum Speichern der ausgewählten Punkte (S210);
Mittel (152), um automatisch und ohne benutzerseitigen Eingriff zu ermitteln, wenn die Ablation an einem aktuellen Punkt der Vielzahl von Punkten beendet ist; und
Mittel (160), um bei der genannten Ermittlung der Beendigung automatisch und ohne benutzerseitigen Eingriff von dem genannten aktuellen Punkt zu einem nächsten Punkt der Vielzahl von Punkten zu lenken (S420);
wobei das Ablationsgerät **dadurch gekennzeichnet ist, dass** es weiterhin Folgendes umfasst:
Mittel (104), um automatisch und ohne benutzerseitigen Eingriff für die Vielzahl von Punkten jeden der folgenden Schritte a) bis d) durchzuführen:
a) Lenken eines Ablationsgeräts zur Annäherung an einen aktuellen der Punkte, um an dem aktuellen Punkt mit der Ablation zu beginnen, wenn der genannte aktuelle Punkt erreicht ist (S410);
b) Ermitteln, ob die genannte Annäherung nicht erfolgreich war, und wenn dies der Fall ist, Speichern eines Orts des genannten aktuellen Punkts (S740);
c) wenn ermittelt wird, dass die genannte Annäherung erfolgreich war, Ermitteln, ob die genannte Ablation an dem genannten aktuellen Punkt nicht erfolgreich war, und wenn dies der Fall ist, Speichern des genannten Orts (S630); und
d) Wiederholen der Schritte a) bis c) für einen nächsten Punkte, bis ein letzter Punkt verarbeitet ist (S430).

2. Ablationsgerät nach Anspruch 1, weiterhin mit Mitteln (120, 104), um automatisch und ohne benutzerseitigen Eingriff eine vorgegebene Bedingung zu detektieren, und mit Mitteln, um bei einer derartigen Detektion a) die genannte Ablation an dem genannten aktuellen Punkt zu unterbrechen, b) die genannte Vorrichtung zu dem nächsten der Vielzahl von Punkten zu bewegen und c) die Ablation an dem genannten nächsten Punkt durchzuführen.

3. Ablationsgerät nach Anspruch 1, das weiterhin Folgendes umfasst:
Mittel (120) zum Erstellen einer elektrophysiologischen Karte eines Herzens, das einer Ablation zu unterziehen ist;
Mittel (104), um mindestens entweder die Morphologie des genannten Herzens zum Erstellen eines Herzmodells zu analysieren oder Spezifikationen eines für die genannte Ablation zu verwendenden Kathetersystems zu liefern, und um basierend auf der erstellten Karte und entsprechend abhängig von mindestens entweder dem gebildeten Modell oder den gelieferten Spezifikationen einen Ablationspfad zu schaffen; und
Mittel (106) zum Aufzeichnen des gebildeten Pfads.

4. Ablationsgerät nach Anspruch 1, das weiterhin Folgendes umfasst:
Mittel (152) zum Detektieren, dass ein Katheter (140), welches die genannte Ablation durchführt, physikalischen Kontakt mit Körpergewebe verloren hat oder dass eine Temperatur an dem genannten Punkt unter einen vorgegebenen Schwellenwert gefallen ist; und
Mittel (104) zum Beenden der Ablation, wenn eine der beiden Situationen eintritt.

5. Ablationsgerät nach Anspruch 1, das weiterhin Folgendes umfasst:
Mittel (144) zum Prüfen, ob eine durch einen elektrischen Stromkreis angetroffene Impedanz innerhalb einer zuvor eingestellten Ablationsdauer unter einen vordefinierten Schwellenwert fällt (S650); und
Mittel (104) zum Beenden der genannten Ablation an dem genannten Punkt, wenn ermittelt wird, dass die Impedanz nicht innerhalb der zuvor eingestellten Dauer unter den genannten vordefinierten Schwellenwert fällt (S630).

6. Ablationsgerät nach Anspruch 1, weiterhin mit Mitteln zum Vergleichen einer erneuten Kartenerstellung nach der Ablation mit einer Kartenerstellung vor der Ablation, wobei das genannte Auftreten aus dem genannten Vergleichen resultiert.

7. Ablationsgerät nach Anspruch 1, weiterhin mit optischer Ausgangssteuerung (128) zum visuellen Scannen entlang eines Pfads, der die genannten neuen Ablationspunkte enthält.

## Revendications

1. Appareil d'ablation, comprenant :
des moyens (120, 104, 106) pour sélectionner une pluralité de points qui résident le long d'un trajet d'ablation prédéterminé dans un tissu d'un corps, et sauvegarder les points sélectionnés (S210) ;
des moyens (152) pour déterminer, automatiquement et sans intervention utilisateur, un instant auquel une ablation est achevée à un point actuel des multiples points ; et
des moyens (160) pour, lors de ladite détermination d'achèvement, réaliser une orientation, automatiquement et sans intervention utilisateur, dudit point actuel à un point suivant des multiples points (S420) ;
dans lequel l'appareil d'ablation est **caractérisé en ce qu'**il comprend en outre :
des moyens (104) pour exécuter, automatiquement et sans intervention utilisateur, pour les multiples points, chacune des étapes suivantes a) à d) :
a) orienter un dispositif d'ablation pour se rapprocher d'un point actuel des points pour le commencement d'une ablation au point actuel lorsque ledit point actuel est atteint (S410) ;
b) déterminer si ledit rapprochement n'est pas réussi, et, dans l'affirmative, stocker un emplacement dudit point actuel (S740) ;
c) s'il est déterminé que ledit rapprochement est réussi, déterminer si ladite ablation audit point actuel n'est pas réussie, et, dans l'affirmative, stocker ledit emplacement (S630) ; et
d) répéter les étapes a) à c) pour un point suivant, jusqu'à ce qu'un dernier point soit traité (S430).

2. Appareil d'ablation selon la revendication 1, comprenant en outre des moyens (120, 104) détectant une condition prédéterminée, automatiquement et sans intervention utilisateur, et des moyens pour, lors d'une telle détection, a) interrompre ladite ablation audit point actuel, b) déplacer ledit dispositif jusqu'au point suivant des multiples points, et c) réaliser l'ablation audit point suivant.

3. Appareil d'ablation selon la revendication 1, comprenant en outre :
des moyens (120) pour réaliser une carte électro-physiologique d'un coeur qui doit subir une ablation ;
des moyens (104) pour analyser une morphologie dudit coeur pour former un modèle du coeur et fournir des spécifications d'un système cathéter destiné à être utilisé dans ladite ablation, ou/et pour créer un trajet d'ablation, en fonction de la carte réalisée et soumis, de façon correspondante, au modèle formé et/ou aux spécifications fournies ; et
des moyens (106) pour enregistrer le trajet formé.

4. Appareil d'ablation selon la revendication 1, comprenant en outre :
des moyens (152) pour détecter qu'un cathéter (140) réalisant ladite ablation a perdu un contact physique avec un tissu corporel ou qu'une température audit point est inférieure à un seuil prédéterminé ; et
des moyens (104) pour terminer une ablation si l'une de ces deux situations se produit.

5. Appareil d'ablation selon la revendication 1, comprenant en outre :
des moyens (144) pour contrôler si une impédance rencontrée par un circuit électrique est inférieure à un seuil prédéfini au sein d'une durée d'ablation préétablie (S650); et
des moyens (104) pour terminer ladite ablation audit point s'il est déterminé que l'impédance n'est pas inférieure audit seuil prédéfini au sein de ladite durée préétablie (S630).

6. Appareil d'ablation selon la revendication 1, comprenant en outre des moyens (104) pour comparer une nouvelle cartographie post ablation à une cartographie pré-ablation, ledit résultat résultant de ladite comparaison.

7. Appareil d'ablation selon la revendication 1, comprenant en outre une commande de sortie optique (128) pour réaliser un balayage visuel le long d'un trajet qui comprend lesdits nouveaux points d'ablation.
